# FASCICULE DE BREVET EUROPEEN

(11) **EP 1 189 627 B1**
(45) Date de publication et mention de la délivrance du brevet: **15.10.2003**
(21) Numéro de dépôt: 00951595.8
(22) Date de dépôt: 16.06.2000
(51) Int. Cl.: A61K 38/17, C07K 14/74, C07K 16/28, A61P 29/00

(54) **COMPOSITIONS CONTENANT DES FORMES SOLUBLES D'HLA-G DANS LE TRAITEMENT DE PATHOLOGIES INFLAMMATOIRES DE LA PEAU**
LÖSLISCHE HLA-G ENTHALTENDE ZUSAMMENSETSUNGEN ZUR BEHANDLUNG ENTZÜNDLICHEN HAUTKRANKHEITEN
COMPOSITIONS CONTAINING SOLUBLE FORMS OF HLA-G FOR TREATING INFLAMMATORY SKIN PATHOLOGIES

(30) Priorité: 18.06.1999 FR 9907736
(43) Date de publication de la demande: 27.03.2002
(73) Titulaire: COMMISSARIAT A L'ENERGIE ATOMIQUE, 75015 Paris (FR)
(72) Inventeur: ARACTINGI, Selim, F-75007 Paris (FR); CAROSELLA, Edgardo, Delfino, F-75016 Paris (FR); DAUSSET, Jean, F-75005 Paris (FR); KHALIL DAHER, Iman, F-94150 Rungis (FR); MOREAU, Philippe, F-91170 Viry-Chatillon (FR); PAUL, Pascale, F-75010 Paris (FR); ROUAS-FREISS, Nathalie, F-75013 Paris (FR)
(74) Mandataire: Vialle-Presles, Marie José
(86) Numéro de dépôt international: FR0001670
(87) Numéro de publication internationale: WO00078337

(56) Documents cités:
- EP-A- 0 677 582
- WO-A-96/31604
- WO-A-98/37098
- ULBRECHT M ET AL: "HLA-G: EXPRESSION IN HUMAN KERATINOCYTES IN VITRO AND IN HUMAN SKIN IN VIVO" EUROPEAN JOURNAL OF IMMUNOLOGY, vol. 24, no. 1, 1994, pages 176-180, XP002007031

## Description

La présente invention est relative à l'utilisation de compositions contenant des formes solubles d'HLA-G dans le traitement de pathologies de la peau et notamment des dermatoses inflammatoires, au procédé d'obtention desdites formes solubles d'HLA-G, ainsi qu'aux anticorps dirigés contre lesdites formes solubles.

Les antigènes du complexe majeur d'histocompatibilité (CMH), se divisent en plusieurs classes, les antigènes de classe I (HLA-A, HLA-B et HLA-C) qui présentent 3 domaines globulaires (α1, α2 et α3), et dont le domaine α3 est associé à la β2 microglobuline, les antigènes de classe II (HLA-DP, HLA-DQ et HLA-DR) et les antigènes de classe III (complément).

Les antigènes de classe I comprennent, outre les antigènes précités, d'autres antigènes, dits antigènes de classe I non classiques, et notamment les antigènes HLA-E, HLA-F et HLA-G; ce dernier, en particulier, est exprimé par les trophoblastes extravilleux du placenta humain normal et les cellules épithéliales thymiques.

La séquence du gène HLA-G (gène HLA-6.0) a été décrite par GERAGHTY et al., (Proc. Natl. Acad. Sci. USA, 1987, 84, 9145-9149) : il comprend 4396 paires de bases et présente une organisation intron/exon homologue à celle des gènes HLA-A, -B et -C. De manière plus précise, ce gène comprend 8 exons, 7 introns et une extrémité non traduite 3'; les 8 exons correspondent respectivement à : exon 1 : séquence signal, exon 2 : domaine extracellulaire α1, exon 3 : domaine extracellulaire α2, exon 4 : domaine extracellulaire α3, exon 5 : région transmembranaire, exon 6 : domaine cytoplasmique I, exon 7 : domaine cytoplasmique II (non traduit), exon 8: domaine cytoplasmique III (non traduit) et région 3' non traduite (GERAGHTY et al., précité; ELLIS et al., J. Immunol., 1990, 144, 731-735; KIRSZENBAUM M. et al., *Oncogeny of hematopoiesis. Aplastic anemia* Eds. E. Gluckman, L. Coulombel, Colloque INSERM/John Libbey Eurotext Ltd). Toutefois le gène HLA-G diffère des autres gènes de classe I, en ce que le codon de terminaison de traduction, en phase, est localisé au niveau du deuxième codon de l'exon 6; en conséquence, la région cytoplasmique de la protéine codée par ce gène HLA-6.0 est considérablement plus courte que celle des régions cytoplasmiques des protéines HLA-A, -B et -C.

Ces antigènes HLA-G sont essentiellement exprimés par les cellules cytotrophoblastiques du placenta et sont considérés comme jouant un rôle dans la protection du foetus (absence de rejet par la mère). En outre, dans la mesure où l'antigène HLA-G est monomorphique, il peut également être impliqué dans la croissance ou la fonction des cellules placentaires (KOVATS et al., Science, 1990, 248, 220-223).

D'autres recherches concernant cet antigène non classique de classe I (ISHITANI et al., *Proc. Natl. Acad. Sci*. USA, 1992, 89, 3947-3951) ont montré que le transcrit primaire du gène HLA-G peut être épissé de plusieurs manières et produit au moins 3 ARNm matures distincts : le transcrit primaire d'HLA-G fournit une copie complète (G1) de 1 200 pb, un fragment de 900 pb (G2) et un fragment de 600 pb (G3).

Le transcrit G1 ne comprend pas l'exon 7 et correspond à la séquence décrite par ELLIS et al. (précité), c'est-à-dire qu'il code une protéine qui comprend une séquence leader, trois domaines externes, une région transmembranaire et une séquence cytoplasmique. L'ARNm G2 ne comprend pas l'exon 3, c'est-à-dire qu'il code une protéine dans laquelle les domaines α1 et α3 sont directement joints ; l'ARNm G3 ne contient ni l'exon 3, ni l'exon 4, c'est-à-dire qu'il code une protéine dans laquelle le domaine α1 et la séquence transmembranaire sont directement joints.

L'épissage qui prévaut pour l'obtention de l'antigène HLA-G2 entraîne la jonction d'une adénine (A) (provenant du domaine codant α1) avec une séquence AC (issue du domaine codant α3), ce qui entraîne la création d'un codon AAC (asparagine) à la place du codon GAC (acide aspartique), rencontré au début de la séquence codant le domaine α3 dans HLA-G1.

L'épissage généré pour l'obtention de HLA-G3 n'entraîne pas la formation d'un nouveau codon dans la zone d'épissage.

Les Auteurs de cet article ont également analysé les différentes protéines exprimées : les 3 ARNm sont traduits en protéine dans la lignée cellulaire .221-G.

Certains des Inventeurs ont montré l'existence d'autres formes épissées d'ARNm d'HLA-G: le transcrit HLA-G4, qui n'inclut pas l'exon 4; le transcrit HLA-G5, qui inclut l'intron 4, entre les exons 4 et 5, provoquant ainsi une modification du cadre de lecture, lors de la traduction de ce transcrit et en particulier l'apparition d'un codon stop, après l'acide aminé 21 de l'intron 4 ; et le transcrit HLA-G6, possédant l'intron 4, mais ayant perdu l'exon 3 (KIRSZENBAUM M. et al., *Proc. Natl. Acad. Sci.* USA, 1994, 91, 4209-4213 ; Demande Européenne EP 0 677 582 ; KIRSZENBAUM M. et al., *Human Immunol.,* 1995, 43, 237-241 ; MOREAU P. et al., *Human Immunol*. 1995, 43, 231-236) ; ils ont également montré que ces différents transcrits sont exprimés dans plusieurs types de cellules humaines foetales et adultes, notamment dans les lymphocytes (KIRSZENBAUM M. et al., *Human Immunol*., 1995, précité ; MOREAU P. et al., *Human Immunol*. 1995, précité).

Il existe donc au moins 6 ARNms HLA-G différents qui codent potentiellement 6 isoformes protéiques d'HLA-G, dont 4 membranaires (HLA-G1, G2, G3 et G4) et 2 solubles (G5, G6).

Il a été montré que l'expression desdites isoformes protéiques d'HLA-G était augmentée par l'interféron γ (Yang et al., J. Immunol., 1996, **156**, 4224-4231).

La fonction immunomodulatrice exercée par ces molécules HLA-G a été décrite et les mécanismes de cette fonction ont été élucidés par la mise en évidence de leur interaction avec des récepteurs inhibiteurs de la lyse présents sur les cellules NK (récepteurs KIR) conduisant à une inhibition des fonctions cytotoxiques de ces cellules ; par exemple N. ROUAS-FREISS et al. (Proc. Nat. Acad. Sci., 1997, **94**, 5249-5254) ont montré que les cellules cibles K562 (lignée érythroleucémique humaine) transfectées par le gène HLA-G étaient protégées de la lyse par les cellules NK. Ces cellules sont habituellement sensible aux cellules NK.

Certains des Inventeurs ont montré que les cellules NK n'expriment aucun transcrit HLA-G (Teyssier et al., Nat. Immunol., 1995, **14,** 262-270), ce résultat confirmant que les produits d'expression du gène HLA-G jouent vraisemblablement un rôle dans les états d'immunotolérance physiologique (grossesse) ou pathologique dans lesquelles les cellules NK sont particulièrement actives (maladies autoimmunes, transplantations) ou sont au contraire inhibées (présence anormale d'HLA-G sur certaines tumeurs ou lors d'infections virales).

Ainsi, certains des Inventeurs ont également montré que certaines tumeurs expriment des molécules HLA-G, ce qui leur permet d'échapper à la surveillance immunitaire (Paul et al., Proc. Nat. Acad. Sci., 1998, **95,** 4510-4515).

L'une des pathologies inflammatoires chronique fréquente est le psoriasis qui est observé chez 2 % des individus des populations caucasiennes. Bien que la maladie se caractérise par une hyperprolifération des kératinocytes de l'épiderme, de très nombreuses études physiopathologiques ont permis de montrer que les lymphocytes T de sous-type Th1, infiltrant le site des lésions et produisant de l'interleukine 2 (IL-2) et de l'interféron γ (IFN γ), jouaient un rôle prépondérant dans la pathogénèse de cette maladie (Z. Bata-Csorgo et al. J. Investigative Dermatol., 1995, 89S-94S ; Schlaak et al., J. Invest. Dermatol., 1994, **102**, 145-149 ; Vogel et al., Eur. J. Biochem., 1995, **227**, 143-149). En effet il a été montré que le surnageant de clones de cellules T issus de lésions de psoriasis était capable d'induire l'hyperprolifération de l'épiderme (Strange et al., J. Invest. Dermatol., 1993, **101,** 695-700). De même, il a été montré que le maintien du phénotype psoriatique de biopsies de peau humaines greffées chez la souris SCID était dépendant de la co-injection des lymphocytes T issus desdites lésions (Gilhar et al., J. Invest. Dermatol., 1997, **109**, 283-288).

L'expression d'HLA-G au niveau des cellules de la peau et son rôle potentiel dans les pathologies associées a été étudié par Ulbrecht et al. (Eur. J. Immunol., 1994, **24**, 176-180). Les auteurs de cet article qui analysent uniquement l'expression des transcrits des isoformes membranaires d'HLA-G et en particulier de l'isoforme dominante HLA-G1 montrent qu'alors qu'un niveau faible de transcrits HLA-G est détecté dans les biopsies de peau présentant des lésions de psoriasis, les transcrits d'HLA-G sont soit absents soit présents à un niveau élevé dans les biopsies de peau saine issues d'individus différents. Ces auteurs concluent donc qu'il n'y a pas de lien évident entre l'expression d'HLA-G et les pathologies de la peau.

Les Inventeurs ont maintenant trouvé que des formes solubles d'HLA-G ont une action thérapeutique sur des états pathologiques inflammatoires de la peau.

La présente invention a pour objet l'utilisation d'une composition essentiellement constituée d'au moins une forme soluble d'HLA-G et d'au moins un véhicule pharmaceutiquement acceptable (excipient), pour la préparation d'un médicament pour le traitement des états pathologiques inflammatoires de la peau.

Les excipients associés à ladite composition sont adaptés à la voie d'administration souhaitée; ils sont sélectionnés parmi les excipients connus de l'homme du métier.

Lesdites HLA-G solubles peuvent avantageusement être administrées par voie générale (voie orale, voie parentérale) ou par voie locale (administration topique).

Dans ce dernier cas, ladite composition se présente sous la forme de crème, de lotion, de liposomes ou de gel.

Les Inventeurs ont maintenant trouvé, de manière inattendue, la présence dans les lésions inflammatoires de la peau, à la fois de macrophages exprimant la protéine HLA-G, localisés au niveau des papilles du derme et de lymphocytes T CD3⁺ infiltrants, exprimant un récepteur inhibiteur des fonctions cytotoxiques, reconnu par HLA-G comme par exemple le récepteur ILT2.

Les Inventeurs ont montré que l'isoforme membranaire dominante HLA-G1 et l'isoforme soluble HLA-G5 sont exprimées uniquement dans les lésions inflammatoires de la peau, alors qu'aucune protéine HLA-G n'est détectée dans la peau saine.

Les Inventeurs ont également montré que la protéine HLA-G et en particulier une isoforme comprenant au moins le domaine α1 d'HLA-G est capable d'inhiber les fonctions de prolifération et les fonctions cytotoxiques des lymphocytes T.

Ainsi, les Inventeurs ont donc mis en évidence le rôle anti-inflammatoire de la protéine HLA-G et en particulier d'une composition constituée d'une forme soluble diffusible comprenant au moins le domaine α1 d'HLA-G comme par exemple l'isoforme HLA-G5, dans le traitement des pathologies inflammatoires de la peau.

Conformément à l'invention, ladite composition est particulièrement adaptée au traitement du psoriasis.

Selon un mode de réalisation avantageux de l'invention, ladite forme soluble d'HLA-G est sélectionnée dans le groupe constitué par les isoformes d'HLA-G solubles comprenant au moins un domaine extracellulaire (α1, α2 ou α3) et les formes solubilisées d'HLA-G1, HLA-G2, HLA-G3 ou HLA-G4 (isoformes membranaires).

Lesdites HLA-G solubles comprennent au moins le domaine extracellulaire α1.

Lesdites formes solubles sont en particulier produites dans un *baculovirus*.

Pour ce qui concerne les formes membranaires, elles sont avantageusement exprimées dans des cellules eucaryotes, conformément au procédé décrit dans la Demande Internationale PCT WO 98/37098, puis solubilisées par traitement de la membrane (agent décapant, tel que la papaïne) et purification convenable, par exemple sur colonne d'immunoaffinité avec des anticorps spécifiques.

On entend par forme soluble d'HLA-G aussi bien les HLA-G solubles (ne comportant pas de domaine transmembranaire) que les HLA-G membranaires solubilisées, par exemple dans les conditions précisées ci-dessus.

De préférence, ladite composition, administrée par voie topique, comprend entre 0,1 et 5 µg/ml, de préférence entre 0,5 et 2,5 µg/ml de forme soluble d'HLA-G.

La présente invention a également pour objet un procédé de préparation d'une HLA-G soluble, caractérisé en ce qu'il comprend les étapes suivantes :
- co-infection de cellules d'insectes par un baculovirus contenant l'ADNc de la β₂M et un autre baculovirus contenant la chaîne α d'une isoforme soluble d'HLA-G;
- culture des cellules d'insectes transfectées, et
- récolte des surnageants et purification de l'isoforme d'HLA-G soluble exprimée.

Selon un mode de mise en oeuvre avantageux dudit procédé, ladite isoforme d'HLA-G soluble est purifiée à l'aide d'un anticorps spécifique des isoformes d'HLA-G solubles.

Selon une disposition avantageuse de ce mode de mise en oeuvre, ledit anticorps est obtenu par immunisation de mammifères non-humains, tels que des lapins avec un peptide immunogène constitué d'un peptide synthétique de 21 acides-aminés, correspondant à la partie C-terminale codée par l'intron 4 des formes solubles HLA-G dont la séquence est SKEGDGGIMSVRESRSLSEDL (SEQ ID NO :1) couplé à la protéine porteuse KLH.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :
- les figures 1 et 2 illustrent la présence des ARN des différentes isoformes d'HLA-G dans les lésions de psoriasis, par comparaison avec la peau saine.
- la figure 3 illustre la présence de l'ARN spécifique de l'isoforme HLA-G5 dans les lésions de psoriasis, par comparaison avec la peau saine.

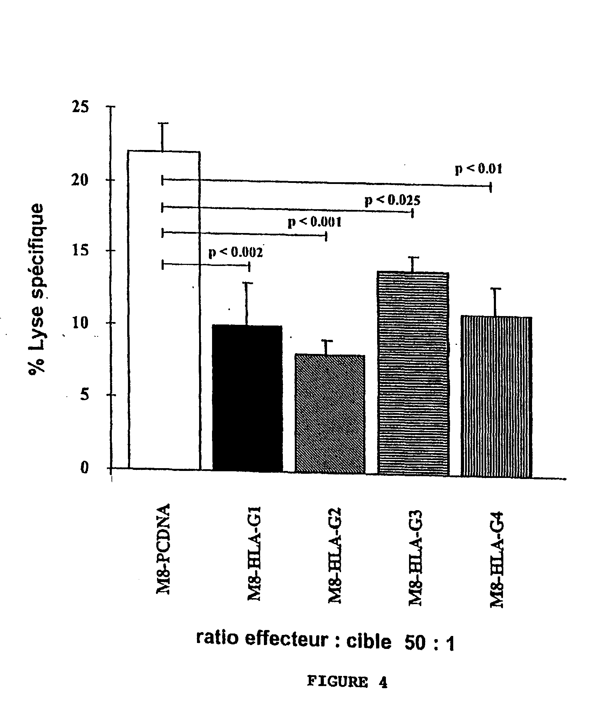

- la figure 4 illustre l'activité inhibitrice des isoformes HLA-G sur des cellules *natural killer* (cellules NK) présentes dans le sang périphérique ; cette figure comporte en abscisse l'isoforme étudiée et en ordonnée, le pourcentage de lyse spécifique. Des cellules M8 (cellules de lignées de mélanome HLA classe I⁺, classe II⁻) transfectées soit avec le vecteur seul (M8-pCDNA) (Invitrogen), soit avec les vecteurs contenant l'ADNc codant HLA-G1 (M8-HLA-G1), l'ADNc codant HLA-G2 (M8-HLA-G2), l'ADNc codant HLA-G3 (M8-HLA-G3), l'ADNc codant HLA-G4 (M8-HLA-G4), sont utilisées comme cibles (T). Des cellules mononucléées de sang périphérique PBMC sont utilisées comme cellules effectrices (E). Les résultats sont exprimés en pourcentage de lyse, enregistré en 4h dans un test de libération du chrome 51 (⁵¹Cr) ;
- la figure 5 illustre l'activité inhibitrice des isoformes HLA-G sur une lignée de lymphocytes T CD8+ restreinte pour HLA-A2 présentant un peptide viral provenant de la matrice du virus de l'influenza, positions 58 à 66; cette figure comporte en abscisse l'isoforme étudiée et en ordonnée, le pourcentage de lyse spécifique ; le ratio cellules effectrices/cellules cibles est de 15:1.

Il doit être bien entendu, toutefois, que ces exemples sont donnés uniquement à titre d'illustration de l'objet de l'invention, dont ils ne constituent en aucune manière une limitation.

### EXEMPLE 1 : Production d'une forme soluble d'une isoforme d'HLA-G chez un baculovirus.

L'isoforme soluble HLA-G5 dont la structure est composée de 3 domaines extracellulaires α1, α2 et α3 associée à la β2-microglobuline (β2M), est issue d'un transcrit alternatif possédant un codon stop dans l'intron 4 du gène HLA-G. La production d'une protéine HLA-G soluble recombinante fait appel à une co-infection de cellules d'insectes par un baculovirus contenant l'ADN complémentaire de la β2M et d'un autre baculovirus contenant l'ADNc de la chaîne α de HLA-G5. Cela permet en effet d'obtenir la forme physiologique HLA-G5/β2M.

### 1. Construction de vecteurs de transfert pour la recombinaison avec le virus BacTen.

### a- Insertion du gène β2M dans un vecteur de transfert :

La séquence d'ADN codante β2M est insérée dans les sites BglII-Kpnl du vecteur de transfert pTen12 (Quantum). Le clone recombinant est vérifié par digestion enzymatique, puis amplifié et stérilisé en vue de la cotransfection avec l'ADN du baculovirus BacTen linéarisé.

### b- Insertion du gène codant HLA-G5 dans un vecteur de transfert :

La séquence codant la molécule HLA-G5 est insérée dans les sites BglII-Kpn1 du vecteur de transfert pTen12. Le clone recombinant est vérifié par digestion enzymatique, puis amplifié et stérilisé en vue de la cotransfection avec l'ADN du baculovirus BacTen (Quantum) linéarisé.

### 2. Construction de baculovirus recombinants.

La première étape consiste à obtenir des baculovirus recombinants HLA-G5A d'une part et β2M d'autre part. Les deux vecteurs de transfert portant les gènes β2M et HLA-G5A sont placés en présence de BacTen linéaire afin de cotransfecter les cellules d'insecte Sf9 en culture. Les surnageants de cotransfection sont récoltés et un clonage par plages de lyse est réalisé pour isoler les clones des baculovirus recombinants. Afin de garantir la qualité de la construction, l'ADN des clones viraux est extrait et l'insertion du gène dans le bon *locus* viral est vérifiée par PCR. Six clones ont été obtenus pour chacune des constructions et chacun a servi à infecter à nouveau des cellules Sf9. Les surnageants de culture ainsi que les cellules ont ensuite été récupérés après centrifugation. Cette protéine est purifiée par immunoaffinité à l'aide des anticorps PAG5-6 (voir exemple 4). La structure de la protéine est vérifiée par *western blot* à la fois avec des anticorps PAG5-6 (anti-HLA-G5) et des anticorps B1G6 (Immunotech) (anti-β2M) spécifiques respectivement de la forme soluble HLA-G5 et de la β2M. Parmi les 6 clones produisant HLA-G5, et les 4 produisant β2M, un a été sélectionné de chaque. Un clone HLA-G5α et un clone β2M ont été utilisés pour coinfecter des cellules Sf9. Par analyse en *western blot* et immunoprécipitation avec un anticorps conformationnel W6/32 (IgG2a, anti-chaînes α de HLA de classe I associées à la β2-m (Sigma)), on peut montrer que le surnageant de cette coinfection contient la protéine HLA-G5 associée à la β2M; elle présente ainsi une forme proche de la forme physiologique. Ces cellules Sf9 permettent donc d'obtenir de grandes quantités de protéine soluble HLA-G5.

### EXEMPLE 2 : Mise en évidence du rôle régulateur d'HLA-G dans les phénomènes T dépendants liés au psoriasis.

### 1. Origine des biopsies de peau

Des biopsies de peau présentant des plaques typiques de lésions de psoriasis chronique, issues de patients n'ayant subi aucun traitement local ou général dans les 15 jours précédant le jour de la biopsie ont été analysées.

Des biopsies de peau saines issues de patientes ayant subi une chirurgie mammaire réductrice sont utilisées comme témoin négatif.

Les échantillons sont séparés en deux, une partie est immédiatement congelée dans l'azote liquide pour l'analyse RT-PCR et la partie restante est incluse dans l'OCT (Miles Inc Diagnostics Division) pour l'analyse immunohistochimique.

### 2. Mise en évidence de l'ensemble des transcrits HLA-G et du transcrit spécifique de l'isoforme HLA-G5 soluble dans les biopsies de peau présentant des lésions de psoriasis

### a) Matériels et méthodes

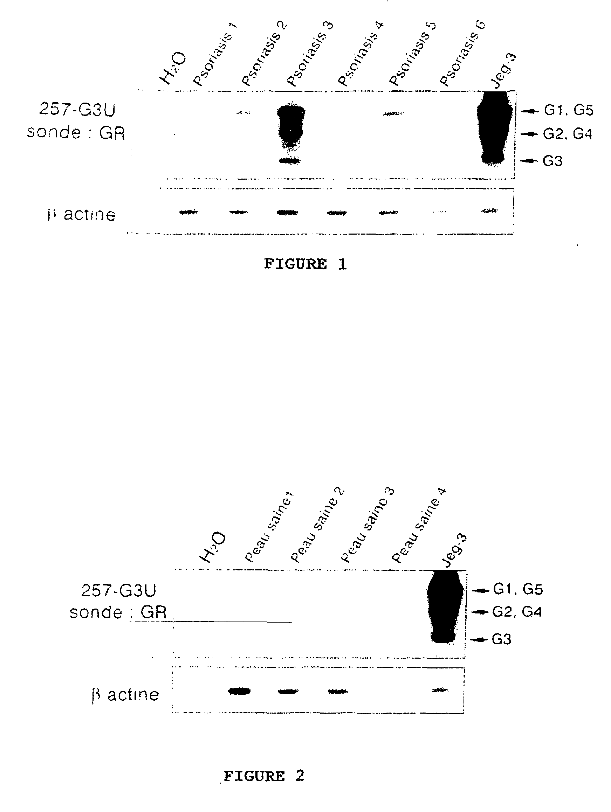

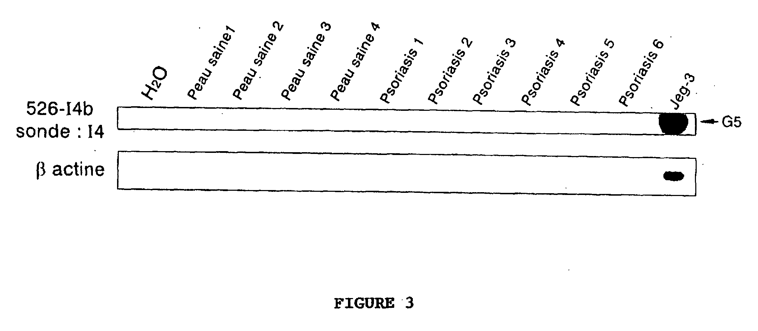

Les ARN messagers sont extraits de biopsies congelées de 6 spécimens de peau lésée de psoriasis (Pso1-Pso-6, figures 1 et 3) et de 4 spécimens de peau normale (Peau saine 1-Peau saine 4, figures 2 et 3) dans les conditions suivantes ; les échantillons sont mis en présence du réactif *RNA Now* (Biogentex, Inc.) et homogénéisés à l'aide de l'homogénéisateur *ultraturrax* (IKA Labortechnic), selon les recommandations du fabricant. La qualité de l'ARN extrait est vérifiée par électrophorèse en gel d'agarose à 1,5%en condition dénaturantes. 5 µg d'ARN sont reverse transcrits en ADNc à l'aide d'une amorce oligo dT de 12 à 18 oligonucléotides (Gibco BRL) et de la transcriptase reverse du virus de Moloney (M-MLV), pendant 1 h à 42°C. Puis, l'ADNc obtenu est amplifié par PCR avec d'une part des amorces reconnaissant l'ensemble des transcrits HLA-G [amorce G.257 (exon 2) et 3G.U (extrémité 3' non-traduite)], utilisées pour l'amplification PCR des transcrits HLA-G correspondants aux différentes isoformes connues d'HLA-G (figures 1 et 2) et d'autre part des amorces spécifiques de la séquence HLA-G5 soluble, à savoir les amorces G.526 et G.i4b (figure 3).

De manière plus précise, les différentes amorces et sondes utilisées sont en conséquence les suivantes :
- G.526 (exon 3) et G3.U (3' UT) pour les isoformes G1, G4 et G5;
- G.526 (exon 3) et G.i4b (intron 4) pour l'isoforme G5 ;
- G.-3 (recouvrant partiellement les exons 2 et 4) et G3.U (3' UT) pour les isoformes G2 et G6;
- G.3-4 (recouvrant partiellement les exons 2 et 5) et G3.U (3' UT) pour l'isoforme G3.

Les conditions de PCR sont les suivantes : 1 min à 90°C, 90 s à 65°C (61°C pour le couple d'amorces G.526 et G.i4b) et 2 min à 72°C, pendant 35 cycles. Les produits PCR sont séparés par électrophorèse en gel d'agarose à 1,5 %.

Les transcrits de la β-actine sont co-amplifiés, pendant 16 cycles, à l'aide d'amorces spécifiques (Clontech) pour normaliser la quantité d'ARN entre les différents échantillons.

Les produits PCR sont ensuite transférés sur une membrane de nylon *(Hybond N*^{*+*}, Amersham), hybridés avec une sonde radiomarquée et l'intensité du signal d'hybridation est quantifié par densitométrie.

Les sondes HLA-G spécifiques sont les suivantes :
- GR spécifique de l'exon 2 qui reconnaît tous les transcrits d'HLA-G, et
- G.I4 F spécifique de l'intron 4, qui ne reconnaît que le transcrit HLA-G5.

Les amorces et les sondes ci-dessus sont décrites dans P. MOREAU et al., C.R. Acad. Sci. Paris, Sciences de la vie/Life Sciences, 1995 ; 318 ; 837-42).

L'ADNc des cellules de choriocarcinome JEG-3 (ATCC), possédant un niveau de transcription élevé d'HLA-G sont utilisés comme témoin positif. Dans les essais ci-dessus, la lignée JEG-3 est cultivée dans un milieu DMEM (Sigma) supplémenté avec du sérum de veau foetal à 10 %, inactivé à la chaleur, des antibiotiques et de la L-glutamine 2 mM. Les lignées cellulaires ne contiennent pas de mycoplasmes.

Les bandes HLA-G spécifiques sont révélées par hybridation avec la sonde GR-spécifique, localisée au niveau de l'exon 2. Les produits de la PCR, co-amplifiés au cours de la même réaction, avec les amorces de la β-actine sont détectés sur la même membrane à l'aide d'une sonde β-actine.

### b) résultats

Les résultats sont illustrés dans les figures 1 à 3.

Les transcrits d'HLA-G sont détectés dans tous les échantillons de peau lésée présentant des lésions de psoriasis et dans seulement 2 des 4 échantillons de peau saine. A l'exception d'un échantillon (échantillon 3), seuls les transcrits correspondant aux isoformes HLA-G1 et HLA-G5 sont détectés dans les échantillons de peau lésée. Dans les biopsies de peau lésée de 6 malades ayant un psoriasis, il existe un niveau transcriptionnel HLA-G plus élevé, en particulier concernant l'isoforme G1/G5 (p<0,05 ; figures 1 et 3).

Dans ces échantillons, le signal de l'isoforme HLA-G5 soluble est absent des 4 spécimens de peaux normales (figure 3), mais retrouvé chez 3 des 6 sujets présentant un psoriasis (figure 3).

L'ensemble des résultats montre que le niveau des transcrits HLA-G1 et G-5 est plus élevé dans les biopsies de peau présentant des lésions de psoriasis que dans les échantillons de peau saine et que le transcrit HLA-G5 est exprimé uniquement dans les biopsies de peau présentant des lésions de psoriasis.

### 3. Mise en évidence de la protéine HLA-G dans les biopsies de peau présentant des lésions de psoriasis

### a) Matériels et méthodes

### a₁) immunohistochimie

Des coupes au cryostat ont été réalisées à partir des échantillons congelés puis les coupes ont été déshydratées dans l'acétone à -20°C pendant 20 min et séchées à l'air libre.

L'immunomarquage est réalisé à l'aide du kit *Dako EnVision+System Peroxidase (AEC),* (Dako) selon les instructions du fabricant.

Les anticorps utilisés sont les suivants :
- 87G et O1G spécifiques d'HLA-G (HLA-G1 à -G5) et 16G1 spécifique d'HLA-G5 (D. Geraghty, Fred Hutchinson Cancer Research Center, Seattle, USA),
- 4H84 spécifique des isoformes d'HLA-G sous forme dénaturée (M. Mc Master, San Francisco, USA) - anti-ILT2 qui reconnaît un KIR impliqué dans la liaison à HLA-G (Navarro et al., Eur. J; Imunol., 1999, 29, 277-283),
- W6/32 : anti antigène de classe I du CMH (Sigma)
- anti-CD3 (Sigma)
- anti CD 14 (Sigma)
- IgG2a de souris : anticorps contrôle (Sigma),

### a₂) double marquage

Les doubles marquages sont réalisés avec les couples d'anticorps suivants :
- 87G et anti-CD68
- 87G et anti-CD11c (Dako)
- anti-ILT2 et anti-CD3

Les conditions utilisées sont les suivantes ; après incubation avec du sérum normal de chèvre, les coupes sont incubées avec le premier anticorps (87G ou anti-ILT2) pendant 60 min, rincées, puis incubées avec un anticorps de chèvre anti-IgG de souris couplé au rouge Texas. Ensuite les coupes sont rincées et incubées pendant 30 min avec le second anticorps couplé à l'isothiocyanate de fluorescéine (FITC) : anticorps anti-CD68 ou anti-CD11c, lorsque le premier anticorps est 87G ou bien anticorps anti-CD3, lorsque le premier anticorps est anti-ILT2.

Dans les coupes contrôles, le premier anticorps (87G ou anti-ILT2) est remplacé par l'anticorps contrôle (IgG2a de souris).

Les coupes sont ensuite analysées en microscopie confocale à laser.

### b) résultats

### b₁) Détection de l'ensemble des protéines HLA-G et de la protéine HLA-G5 soluble dans les biopsies de peau

En utilisant l'anticorps 87G ou l'anticorps O1G qui reconnaissent toutes les isoformes d'HLA-G, aucune expression de la protéine HLA-G n'est détectée dans les échantillons de peau saine.

En revanche, dans les 9 échantillons de peau présentant des lésions de psoriasis, l'expression d'HLA-G est détectée dans des cellules des papilles du derme. Le niveau d'expression d'HLA-G est variable selon les échantillons ; il est élevé dans certains échantillons. De plus, dans deux échantillons seulement, l'expression d'HLA-G est également détectée dans l'épiderme, dans des zones proches de l'infiltrat de cellules inflammatoires du derme, soit au niveau de foyers isolés de cellules inflammatoires ou bien dans quelques kératinocytes. La localisation d'HLA-G au niveau de ces quelques kératinocytes est vraisemblablement liée à la diffusion de l'isoforme HLA-G5 à partir des cellules du derme exprimant ladite isoforme.

En utilisant l'anticorps 16G1 qui reconnaît spécifiquement l'isoforme HLA-G5 soluble, un marquage spécifique des cellules des papilles du derme est également détecté.

### b₂) Localisation des cellules exprimant HLA-G dans les lésions de psoriasis

Les doubles marquages avec l'anticorps 87G et l'anticorps anti-CD3 ou l'anticorps anti-CD14 montrent qu'HLA-G est co-localisée au niveau des cellules exprimant CD14, en revanche aucune co-localisation d'HLA-G avec les lymphocytes T (CD3⁺) n'est observée.

### b₃) Les cellules exprimant HLA-G sont des macrophages CD68⁺, CD11c⁺

Le marquage des coupes sériées des échantillons de peau présentant des lésions de psoriasis avec les anticorps 87G et anti-CD68 ou anti-CD11c qui reconnaissent spécifiquement les macrophages, démontrent que la quasi-totalité des cellules qui expriment HLA-G expriment aussi CD68 et CD11c et sont donc des macrophages.

### b₄) Le récepteur KIR de type ILT2 est présent dans les lymphocytes T infiltrant des lésions de psoriasis

Le marquage des biopsies de peau présentant des lésions de psoriasis avec l'anticorps anti-ILT2 montre la présence d'un infiltrat dense au niveau du derme superficiel. En revanche aucun marquage n'est détecté dans le derme des échantillons de peau saine. De plus, des expériences de double marquage avec les anticorps anti-CD3 et anti-ILT2 montre la présence de quelques cellules doublement marquées au niveau des papilles du derme.

En résumé, l'ensemble des résultats présentés ci-dessus montrent que :
- dans la peau saine l'expression des ARN HLA-G est faible ou indétectable et la protéine HLA-G est absente,
- dans les lésions de psoriasis il existe une expression accrue des ARN et de la protéine des isoformes HLA-G1 et -G5,
- dans les lésions de psoriasis l'expression d'HLA-G est localisée au niveau de macrophages et
- dans les lésions de psoriasis, les lymphocytes T infiltrants expriment un récepteur inhibiteur des fonctions cytotoxique reconnu par HLA-G (récepteur ILT2).

Ces résultats montrent la présence au niveau des lésions de psoriasis, à la fois de cellules exprimant HLA-G (macrophages des papilles du derme) et de cellules infiltrantes responsables des lésions de psoriasis (lymphocytes T CD3⁺), exprimant un récepteur inhibiteur des fonctions cytotoxique reconnu par HLA-G (récepteur ILT2).

En conséquence ces résultats démontrent le rôle direct d'HLA-G et en particulier de l'isoforme membranaire prédominante HLA-G1 et de l'isoforme soluble diffusible HLA-G5 dans la régulation locale des phénomènes T dépendants responsables du psoriasis.

### EXEMPLE 3 : Rôle du domaine extracellulaire α1 d'HLA-G dans l'inhibition des fonctions immunes et application dans le traitement du psoriasis

### 1. Rôle du domaine extracellulaire α1 d'HLA-G dans l'inhibition de l'activité cytotoxique des cellules NK -et des cellules T

### a) cellules NK

L'utilisation de cellules transfectées avec chacune des isoformes HLA-G1, -G2, -G3 ou -G4 en tant que cellules cibles face à des cellules immunocompétentes *natural killer* présentes dans le sang périphérique permet de démontrer que chacune des isoformes est capable d'inhiber l'activité cytotoxique des cellules *natural killer* (Fig. 4). Ces expériences ont été effectuées chez plus de dix donneurs volontaires sains et la significativité de l'inhibition exercée par chacune des isoformes a été validée par des tests statistiques (Fig. 4).

### b) cellules T

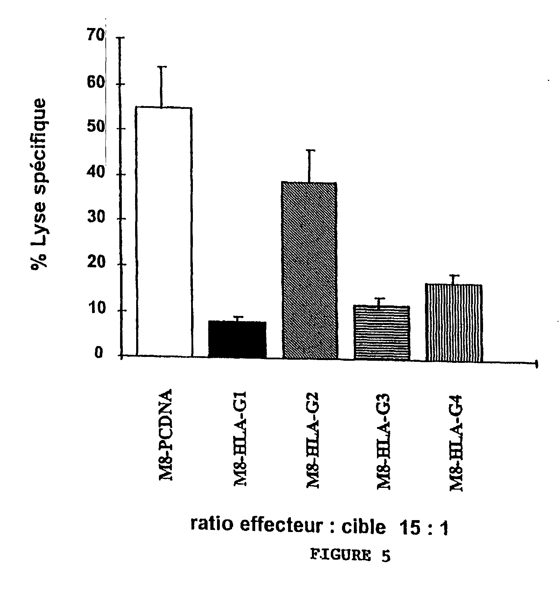

Des tests similaires de cytotoxicité *in vitro* réalisés avec les mêmes cellules cibles faces à des cellules T CD8+ restreintes par le CMH et spécifiques d'un peptide antigénique ont également démontré que chacune des isoformes HLA-G1, -G2, -G3 et -G4 inhibe de façon significative l'activité cytotoxique de ces cellules T (Fig. 5).

Sur la base de la structure de l'isoforme HLA-G3 constituée uniquement du domaine extracellulaire α1 possédant toutes les propriétés inhibitrices décrites ci-dessus, on peut conclure à la fonctionnalité de ce domaine. Ce domaine contient donc le motif fonctionnel de HLA-G et pourra donc être utilisé comme agent pharmacologique en vue d'une immunotolérance.

### 2. Application dans le traitement du psoriasis

Le rôle direct d'HLA-G et en particulier de l'isoforme membranaire prédominante HLA-G1 et de l'isoforme soluble diffusible HLA-G5 dans la régulation locale des phénomènes T dépendants responsables du psoriasis a été démontrée à l'exemple 2.

Le rôle d'HLA-G et en particulier du domaine α1 desdites isoformes dans l'inhibition des fonctions T a été démontrée ci-dessus.

En conséquence, l'ensemble des résultats indiquent qu'une composition pharmaceutique contenant ladite isoforme HLA-G en particulier sous forme soluble diffusible, comme l'isoforme HLA-G5, présente un rôle protecteur dans le traitement du psoriasis.

### EXEMPLE 4 : Production d'un anticorps nommé PAG5-6 reconnaissant spécifiquement les formes solubles de HLA-G (HLA-G5 et HLA-G6) sous forme d'un sérum polyclonal obtenu chez le lapin.

L'immunisation de lapins avec un peptide immunogène constitué d'un peptide synthétique de 21 acides-aminés, correspondant à la partie C-terminale codée par l'intron 4 des formes solubles HLA-G dont la séquence est SKEGDGGIMSVRESRSLSEDL (SEQ ID NO:1) couplé à la protéine porteuse KLH, permet d'obtenir un sérum polyclonal reconnaissant spécifiquement les formes solubles de HLA-G (HLA-G5 et HLA-G6) par les techniques d'immunoprécipitation, immunoempreinte (Western Blot), immunohistochimie et immunoenzymatique type ELISA. Ce sérum a été purifié sur colonne d'affinité (protéine G-Sépharose) et peut à la fois servir à la détection, la titration, et la purification des formes solubles HLA-G.

## Revendications

1. Utilisation d'une composition essentiellement constituée d'au moins une forme soluble d'HLA-G et d'au moins un véhicule pharmaceutiquement acceptable, pour la préparation d'un médicament pour le traitement des états pathologiques inflammatoires de la peau.

2. Utilisation selon la revendication 1, **caractérisée en ce que** ladite forme soluble d'HLA-G est sélectionnée dans le groupe constitué par les isoformes d'HLA-G solubles comprenant au moins le domaine extracellulaire α1 et les formes solubilisées d'HLA-G1, HLA-G2, HLA-G3 ou HLA-G4.

3. Utilisation selon la revendication 1 ou la revendication 2, **caractérisée en ce que** ladite composition comprend entre 0,1 et 5 µg/ml, de préférence entre 0,5 et 2,5 µg/ml de forme soluble d'HLA-G.

4. Procédé de préparation d'une HLA-G soluble, **caractérisé en ce qu'**il comprend les étapes suivantes :
- co-infection de cellules d'insectes par un baculovirus contenant l'ADNc de la β₂M et un autre baculovirus contenant la chaîne α d'une isoforme d'HLA-G soluble ;
- culture des cellules d'insectes transfectées, et
- récolte des surnageants et purification de l'isoforme d'HLA-G soluble exprimée.

5. Procédé selon la revendication 4, **caractérisé en ce que** ladite isoforme d'HLA-G soluble est purifiée à l'aide d'un anticorps spécifique des isoformes d'HLA-G soluble.

6. Procédé selon la revendication 5, **caractérisé en ce que** ledit anticorps est obtenu par immunisation de mammifères non-humains, notamment des lapins avec un peptide immunogène de SEQ ID NO:1 couplé à la protéine porteuse KLH.

7. Anticorps anti-HLA-G soluble, **caractérisé en ce qu'**il est obtenu par immunisation de mammifères non-humains, notamment des lapins avec un peptide immunogène constitué d'un peptide synthétique de 21 acides-aminés de SEQ ID NO:1 couplé à la protéine porteuse KLH.

## Patentansprüche

1. Verwendung einer Zusammensetzung, die im wesentlichen aus wenigstens einer löslichen Form von HLA-G und wenigstens einem pharmazeutisch verträglichen Trägerstoff besteht, zur Herstellung eines Medikaments zur Behandlung von entzündlichen pathologischen Zuständen der Haut.

2. Verwendung nach Anspruch 1, **dadurch gekennzeichnet, daß** die lösliche Form von HLA-G ausgewählt ist aus der Gruppe bestehend aus den löslichen HLA-G-Isoformen, die wenigstens die extrazelluläre α1-Domäne umfassen, und den solubilisierten Formen von HLA-G1 HLA-G2, HLA-G3 oder HLA-G4.

3. Verwendung nach Anspruch 1 oder Anspruch 2, **dadurch gekennzeichnet, daß** die Zusammensetzung zwischen 0,1 und 5 µg/ml, vorzugsweise zwischen 0,5 und 2,5 µg/ml lösliche Form von HLA-G umfaßt.

4. Verfahren zur Herstellung eines löslichen HLA-G, **dadurch gekennzeichnet, daß** es die folgenden Schritte umfaßt:
- Coinfektion von Insektenzellen mit einem Baculovirus, das die DNA für das β₂M enthält, und einem weiteren Baculovirus, das die α-Kette einer löslichen HLA-G-Isoform enthält;
- Kultivierung der transfizierten Insektenzellen, und
- Ernte der Überstände und Reinigung der exprimierten löslichen HLA-G-Isoform.

5. Verfahren nach Anspruch 4, **dadurch gekennzeichnet, daß** die lösliche HLA-G-Isoform mit Hilfe eines für die Isoformen von löslichem HLA-G spezifischen Antikörpers gereinigt wird.

6. Verfahren nach Anspruch 5, **dadurch gekennzeichnet, daß** der Antikörper durch Immunisierung von nichthumanen Säugern, insbesondere Kaninchen, mit einem immunogenen Peptid gemäß SEQ ID NO:1, das an das Trägerprotein KLH gekoppelt ist, erhalten wird.

7. Antikörper gegen lösliches HLA-G, **dadurch gekennzeichnet, daß** er durch Immunisierung von nichthumanen Säugern, insbesondere Kaninchen, mit einem immunogenen Peptid erhalten ist, das aus einem synthetischen Peptid mit 21 Aminosäuren gemäß SEQ ID NO:1 besteht, das an das Trägerprotein KLH gekoppelt ist.

## Claims

1. The use of a composition essentially consisting of at least one soluble form of HLA-G and of at least one pharmaceutically acceptable vehicle, for preparing a medicinal product for treating inflammatory pathological skin conditions.

2. The use as claimed in claim 1, **characterized in that** said soluble form of HLA-G is selected from the group consisting of the soluble isoforms of HLA-G comprising at least the α1 extracellular domain and the solubilized forms of HLA-G1, HLA-G2, HLA-G3 or HLA-G4.

3. The use as claimed in claim 1 or claim 2, **characterized in that** said composition comprises between 0.1 and 5 µg/ml, preferably between 0.5 and 2.5 µg/ml, of soluble form of HLA-G.

4. A method for preparing a soluble HLA-G, **characterized in that** it comprises the following steps:
- coinfecting insect cells with a baculovirus containing the β₂M cDNA and another baculovirus containing the α chain of a soluble isoform of HLA-G;
- culturing the transfected insect cells, and
- harvesting the supernatants and purifying the soluble isoform of HLA-G expressed.

5. The method as claimed in claim 4, **characterized in that** said soluble isoform of HLA-G is purified using an antibody specific for the soluble isoforms of HLA-G.

6. The method as claimed in claim 5, **characterized in that** said antibody is obtained by immunizing nonhuman mammals, in particular rabbits, with an immunogenic peptide of SEQ ID NO:1 coupled to the KLH carrier protein.

7. An anti-soluble HLA-G antibody, **characterized in that** it is obtained by immunizing nonhuman mammals, in particular rabbits, with an immunogenic peptide consisting of a 21 amino acid synthetic peptide of SEQ ID No:1 coupled to the KLH carrier protein.
